# EUROPEAN PATENT SPECIFICATION

(11) **EP 2 170 333 B1**
(45) Date of publication and mention of the grant of the patent: **20.02.2013**
(21) Application number: 08732776.3
(22) Date of filing: 24.03.2008
(51) Int. Cl.: A61K 31/495, A61P 9/10, G01N 33/68, A61K 9/08, A61K 9/22

(54) **RANOLAZINE FOR ELEVATED BRAIN-TYPE NATRIURETIC PEPTIDE**
RANOLAZIN FÜR ERHÖHTES NATRIURETISCHES PEPTID VOM GEHIRN-TYP
UTILISATION DE RANOLAZINE POUR PEPTIDE CÉRÉBRAL NATRIURÉTIQUE ÉLEVÉ

(30) Priority: 31.05.2007 US 941210 P
(43) Date of publication of application: 07.04.2010
(73) Proprietor: Gilead Sciences, Inc., Foster City, CA 94404 (US)
(72) Inventor: BLACKBURN, Brent, Los Altos, CA 94022 (US); JERLING, Marcus, S-167 56 Bromma (SE); WOLFF, Andrew, San Francisco, CA 94133 (US)
(74) Representative: Schnappauf, Georg
(86) International application number: PCT/US2008/058063
(87) International publication number: WO 2008/150565

(56) References cited:
- WO-A2-00/13687
- HEART PROTECTION STUDY COLLABORATIVE GROUP: "N-Terminal Pro-B-Type Natriuretic Peptide, Vascular Disease Risk, and Cholesterol Reduction Among 20,536 Patients in the MRC/BHF Heart Protection Study" JOURNAL OF THE AMERICAN COLLEGE OF CARDIOLOGY, ELSEVIER, NEW YORK, NY, US, vol. 49, no. 3, 17 January 2007 (2007-01-17), pages 311-319, XP005834244 ISSN: 0735-1097
- MORROW DAVID A ET AL: "Evaluation of B-type natriuretic peptide for risk assessment in unstable angina/non-ST-elevation myocardial infarction: B-type natriuretic peptide and prognosis in TACTICS-TIMI 18" JOURNAL OF THE AMERICAN COLLEGE OF CARDIOLOGY, ELSEVIER, NEW YORK, NY, US, vol. 41, no. 8, 16 April 2003 (2003-04-16), pages 1264-1272, XP002484190 ISSN: 0735-1097
- JERNBERG TOMAS ET AL: "N-terminal pro brain natriuretic peptide on admission for early risk stratification of patients with chest pain and no ST-segment elevation" JOURNAL OF THE AMERICAN COLLEGE OF CARDIOLOGY, vol. 40, no. 3, 7 August 2002 (2002-08-07), pages 437-445, XP002510229 ISSN: 0735-1097
- MORROW DAVID A ET AL: "Effects of ranolazine on recurrent cardiovascular events in patients with non-ST-elevation acute coronary syndromes - The MERLIN-TIMI 36 randomized trial" JAMA THE JOURNAL OF THE AMERICAN MEDICAL ASSOCIATION, AMERICAN MEDICAL ASSOCIATION, US, vol. 297, no. 16, 25 April 2007 (2007-04-25), pages 1775-1783;E1, XP002480268 ISSN: 0098-7484 cited in the application
- MORROW DAVID A ET AL: "B-type natriuretic peptide and the effect of ranolazine in patients with non-ST elevation acute coronary syndromes in the MERLIN-TIMI 36 trial" CIRCULATION, AMERICAN HEART ASSOCIATION, DALLAS, TX, vol. 116, no. 16, Suppl. S, 1 October 2007 (2007-10-01), page 382, XP008092823 ISSN: 0009-7322
- MEGA JESSICA L ET AL: "Anti-ischemic effects of ranolazine in women: Results from the randomized, placebo-controlled MERLIN-TIMI 36 trial" CIRCULATION, AMERICAN HEART ASSOCIATION, DALLAS, TX, vol. 116, no. 16, Suppl. S, 1 October 2007 (2007-10-01), page 539, XP008091380 ISSN: 0009-7322
- NEWBY L KRISTIN ET AL: "Does ranolazine have a place in the treatment of acute coronary syndromes?", JAMA : THE JOURNAL OF THE AMERICAN MEDICAL ASSOCIATION, AMERICAN MEDICAL ASSOCIATION, UNITED STATES, vol. 297, no. 16, 25 April 2007 (2007-04-25), pages 1824-1825, XP008100304, ISSN: 1538-3598
- STANLEY W C: "RANOLAZINE: NEW APPROACH FOR THE TREATMENT OF STABLE ANGINA PECTORIS", EXPERT REVIEW OF CARDIOVASCULAR THERAPY, FUTURE DRUGS, LONDON, GB LNKD- DOI:10.1586/14779072.3.5.821, vol. 3, no. 5, 1 September 2005 (2005-09-01), pages 821-829, XP009062744, ISSN: 1477-9072

## Description

### FIELD OF THE INVENTION

Disclosed is identifying the level of natriuretic peptides, such as brain-type natriuretic peptide (BNP) and N-terminal pro-brain natriuretic peptide (NT-proBNP). in the blood plasma of a mammalian patient and, if the level of BNP is ≥ 80 pg/ml blood administering ranolazine to the patient. The invention is directed to ranolazine for use in a method for reducing the risk of adverse coronary event including, coronary artery disease (CAD), cardiovascular death, myocardial infarction, acute heart failure, ischemia, recurrent ischemia, acute coronary syndrome and diastolic dysfunction, a mamalian in patient with a plasma BNP of 80 pg/mL blood or greater.

### BACKGROUND OF THE INVENTION

The natriuretic peptide system consists of structurally similar natriuretic peptides that compensate for changes in volume and pressure by promoting the removal of sodium ions from the blood. Elevated levels of arterial natriuretic peptide (ANP) and BNP may be found in patients with coronary disease. Clinical studies have indicated that the testing for elevated levels of BNP or NT-proBNP facilitates the diagnosis of heart failure and is validated by biomarker for increased risk. Persson, et al. "Diastolic Dysfunction in Heart Failure with Preserved Systolic Function: Need for Object Evidence" JACC. 49(6):687-694 (2007); Bibbins-Domingo et al. "N-Terminal Fragment of the Prohormone Brain-Type Natriuretic Peptide (NT-proBNP). Cardiovascular Events, and Mortality in Patients with Stable Coronary Heart Disease" JAMA, 297(2):169-176(2007).

Ranolazine, a late I_{Na} inhibitor, currently available in the U.S. under the trade name Ranexa® for angina, is suitable for delivery in the form of an oral dose, such as a compressed tablet, or an intravenous dose.

U.S. Patent No. 4,567,264, discloses ranolazine, (±)-N-(2,6-dimethylphenyl)-4-[2-hydroxy-3-(2-methoxyphenoxy)-propyl]-1-piperazineacetamide, and its pharmaceutically acceptable salts, and their use in the treatment of cardiovascular diseases, including arrhythmias, variant and exercise-induced angina, and myocardial infarction. In its dihydrochloride salt form, ranolazine is represented by the formula:

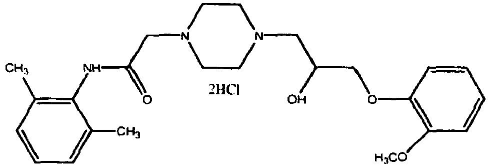

U.S. Patent No. 5,506,229, discloses the use of ranolazine and its pharmaceutically acceptable salts and esters for the treatment of tissues experiencing a physical or chemical insult, including cardioplegia, hypoxic or reperfusion injury to cardiac or skeletal muscle or brain tissue, and for use in transplants. Oral and parenteral formulations are disclosed, including controlled release formulations. In particular, Example 7D of U.S. Patent No. 5,506,229 describes a controlled release formulation in capsule form comprising microspheres of ranolazine and microcrystalline cellulose coated with release controlling polymers. This patent also discloses IV ranolazine formulations which at the low end comprise 5 mg ranolazine per milliliter of an IV solution containing about 5% by weight dextrose.

The presently preferred route of administration for ranolazine and its pharmaceutically acceptable salts is oral. A typical oral dosage form is a compressed tablet, a hard gelatin capsule filled with a powder mix or granulate, or a soft gelatin capsule (softgel) filled with a solution or suspension. U.S. Patent No. 5,472,707. , discloses a high-dose oral formulation employing supercooled liquid ranolazine as a fill solution for a hard gelatin capsule or softgel.

U.S. Patent Application Publication Number 2006/0177502, discloses oral sustained release dosage forms in which the ranolazine is present in 35-50%, preferably 40-45% ranolazine. In one embodiment the ranolazine sustained release formulations of the invention include a pH dependent binder, a pH independent binder; and one or more pharmaceutically acceptable excipients. Suitable pH dependent binders include, a methacrylic acid copolymer, for example Eudragit* (Eudragit® L100-55, pseudolatex of Eudragit® L100-55, ) partially neutralized with a strong base, for example, sodium hydroxide, potassium hydroxide, or ammonium hydroxide, in a quantity sufficient to neutralize the methacrylic acid copolymer to an extent of 1-20%, for example 3-6%. Suitable pH independent binders include, , hydroxypropylmethylcellulose (HPMC), for example Methocel® E10M Premium CR grade HPMC or Methocel® E4M Premium HPMC. Suitable pharmaceutically acceptable excipients include magnesium stearate and microcrystalline cellulose (Avicel® pH 101).

### SUMMARY OF THE INVENTION

The present invention is defined in the appended claims. Subject-matters which are not encompassed by the scope of the claims do not form part of the present invention.

It has been discovered that ranolazine, a late I_{Na} current inhibitor, is capable of reducing the risk of an adverse coronary event, including, coronary artery disease (CAD), cardiovascular death, myocardial infarction, acute heart failure, ischemia. recurrent ischemia, acute coronary syndrome, diastolic dysfunction, in a patient exhibiting elevated levels of natriuretic peptides. By identifying the level of BNP in a patient suffering from a coronary disease, and, if the level of BNP is 80 pg/mL or greater, it has been found that administering ranolazine reduces the risk of occurrence of an adverse coronary event in such a patient. Further, by administering ranolazine to a patient with a BNP level of 80 pg/mL or greater, it has been found that adverse coronary events related to diastolic dysfunction and/or acute coronary syndrome are reduced.

One embodiment of the invention is directed to ranolazine for use in amethod of reducing the risk of an adverse coronary event, including, coronary artery disease (CAD), cardiovascular death, myocardial infarction, acute heart failure, ischemia, recurrent ischemia, acute coronary syndrome, and diastolic dysfunction, in a mamalian patient exhibiting 80 pg/ml or greater of a natriuretic peptide in blood the method comprising identifying a patient exhibiting elevated levels of a natriuretic peptide; and administering the patient a therapeutically effective amount of ranolazine. The ranolazine may be administered as an oral dose or as an IV solution. In some cases, the patient may be administered an IV solution followed by an oral dose. Preferably, when administered as an oral dose, ranolazine is administered in a sustained release tablet.

In one embodiment, the elevated levels of natriuretic peptide are correlated with higher risks of adverse coronary events in a patient arising from diseases including, coronary artery disease (CAD), cardiovascular death, myocardial infarction, acute heart failure, ischemia, recurrent ischemia, acute coronary syndrome, diastolic dysfunction.

In one embodiment, the natriuretic peptide is BNP or NT-proBNP and the elevated levels are 80 picograms or greater of natriuretic peptide per milliliter of blood.

In another embodiment, the invention is directed to kits of parts comprising an assay that detects levels of natriuretic peptides in blood plasma and/or heart tissue of a mammalian patient and a pharmaceutical dosage of ranulazine. The pharmaceutical dosage may in the form of an IV solution and/or an oral dose. The oral dose may be in the form of one or more sustained release tablets.

### DESCRIPTION OF THE DRAWINGS

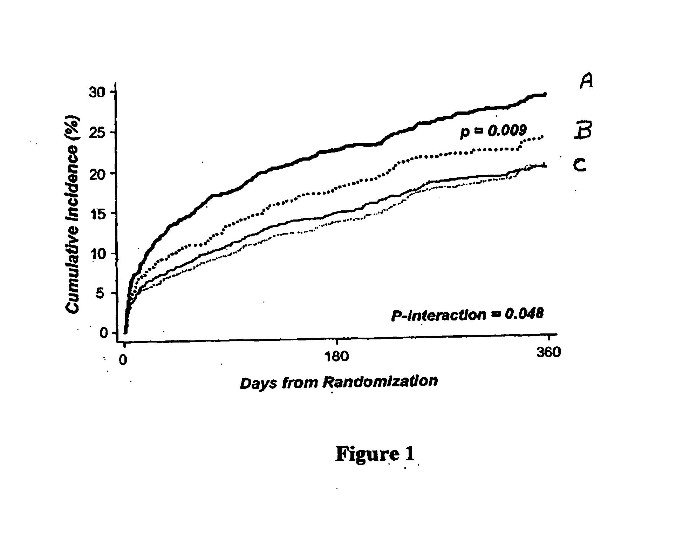

Figure 1 shows the cumulative incidence (%) of cardiovascular death, myocardial infarction, or recurrent ischemia for patients with positive BNP on placebo (curve A), with positive BNP on ranolazine (curve B), or negative BNP and placebo or ranolazine administered (2 curves C) versus days from randomization.

### DETAILED DESCRIPTION OF THE INVENTION

As noted above, disclosed are methods of reducing the risk of an adverse coronary event, including, coronary artery disease (CAD), cardiovascular death, myocardial infarction, acute heart failure, ischemia, recurrent ischemia, acute coronary syndrome, diastolic dysfunction, in a patient exhibiting elevated levels of natriuretic peptides comprising administering ranolazine to these patients. However, prior to describing this invention in more detail, the following terms will first be defined.

### 1. Definitions

In this specification and in the claims that follow, reference will be made to a number of terms that shall be defined to have the following meanings.

The term "natriuretic peptide" refers to peptides that causes natriuresis, the excretion of an excessively large amount of sodium in the urine. The natriuretic peptides are produced by the heart and vasculature:
A-type natriuretic peptide (or "ANP") is secreted largely by the atrial myocardium in response to dilatation.
B-type natriuretic peptide (or "BNP") is produced mainly by the ventricular myocardium.
C-type natriuretic peptide (or "CNP") is produced by endothelial cells that line the blood vessels.

Preferably the natriuretic peptide is "BNP" and "NT-proBNP". Both arise from a 108-amino acid pro form synthesized in the ventricles. In response to pressure overload, the pro form is cleaved into BNP (32 amino acids) and NT-proBNP (78 amino acids). Once released into the circulation, BNP plays a homeostatic role as a systemic vasodilator and diuretic. NT-proBNP appears to be a by-product. Of special interest for laboratory medicine, BNP has a shorter half-life than NT-proBNP. Thus, BNP fluctuates on a faster timescale, while NT-proBNP remains more stable in blood samples. Levels of BNP and NT-proBNP may be tested using commercially available assays as discussed below.

"Elevated levels of natriuretic peptide" refers to a concentration of endogenous natriuretic peptide that is predictive of a future adverse coronary event in the patient. For example, in the case of BNP, elevated levels refers to 80 picograms (pg) or greater per milliliter of blood, preferably, 100 picograms or greater of natriuretic peptide per milliliter of blood. In another instance, the BNP concentration is between 100 and 300 picograms of natriuretic peptide per milliliter of blood. In another instance, the BNP concentration is between 300 and 600 picograms of natriuretic peptide per milliliter of blood. In another instance, the BNP concentration is between 600 and 900 picograms of natriuretic peptide per milliliter of blood. In another instance, the BNP concentration is greater than 900 picograms of natriuretic peptide per milliliter of blood. It has been found that identifying an elevated level of natriuretic peptide in a coronary patient and administering ranolazine to the patient reduces the risk of a future adverse coronary event in the patient.

An "adverse coronary event" or "adverse cardiac event" refers to a non-incremental decrease in the coronary performance of the patient due to a coronary or cardiac disease which event may involve either morbidity or hospitalization. Such adverse coronary/cardiac events include, by way of example, coronary artery disease ("CAD"), cardiovascular death (CD), myocardial infarction (MI), acute heart failure, ischemia, recurrent ischemia (RI), acute coronary syndrome (ACS), diastolic dysfunction.

"Ranolazine" is the compound (±)-N-(2,6-dimethylphenyl)-4-[2-hydroxy-3-(2-methoxyphenoxy)propyl]-1-piperazine-acetamide, and its pharmaceutically acceptable salts, and mixtures thereof. Unless otherwise stated the ranolazine plasma concentrations used in the specification and examples refer to ranolazine free base. At pH of, in an aqueous solution titrated with hydrogen chloride, ranolazine will be present in large part as its dihydrochloride salt.

"Physiologically acceptable pH" refers to the pH of an intravenous solution which is compatible for delivery into a human patient. Preferably, physiologically acceptable pH's range is from 4 to 8.5 and preferably from 4 to 7. Without being limited by any theory, the use of intravenous solutions having a pH of 4 to 6 are deemed physiologically acceptable as the large volume of blood in the body effectively buffers these intravenous solutions.

"Coronary diseases" or "cardiovascular diseases" refer to diseases of the cardiovasculature arising from any one or more than one of, for example, heart failure, including congestive heart failure, acute heart failure, ischemia, recurrent ischemia, myocardial infarction, arrhythmias, angina (including exercise-induced angina, variant angina, stable angina, unstable angina), acute coronary syndrome, diabetes, and intermittent claudication. The treatment of such disease states is disclosed in various U.S. patents and patent applications, including U.S. Patent Nos. 6,503,911 and 6,528,511, U.S. Patent Application Serial Nos. 2003/0220344 and 2004/0063717.

"Acute coronary syndrome" or "ACS" refers to a range of acute myocardial ischemic states. It encompasses unstable angina and non-ST-segment elevation myocardial infarction (UA/NSTEMI), and ST segment elevation myocardial infarction (STEMI).

The term "diastolic dysfunction" refers to and is characterized by an increase in diastolic filling pressure, which may be responsible for the occurrence of dyspnea. This symptom may occur during exercise ("latent" diastolic dysfunction) or may be present also at rest ("manifest" diastolic dysfunction.

"Optional" and "optionally" mean that the subsequently described event or circumstance may or may not occur, and that the description includes instances where the event or circumstance occurs and instances in which it does not. For example, "optional pharmaceutical excipients" indicates that a formulation so described may or may not include pharmaceutical excipients other than those specifically stated to be present, and that the formulation so described includes instances in which the optional excipients are present and instances in which they are not.

"Treating" and "treatment" refer to any treatment of a disease in a patient and include: preventing the disease from occurring in a subject which may be predisposed to the disease but has not yet been diagnosed as having it; inhibiting the disease, i.e., arresting its further development; inhibiting the symptoms of the disease; relieving the disease, i.e., causing regression of the disease, or relieving the symptoms of the disease. The "patient" is a mammal, preferably a human.

The term "therapeutically effective amount" refers to that amount of ranolazine that is sufficient to effect treatment, as defined above, when administered to a mammal in need of such treatment. The therapeutically effective amount will vary depending upon the subject and disease condition being treated, the weight and age of the subject, the severity of the disease condition, the manner of administration which can readily be determined by one of ordinary skill in the art.

"Immediate release" ("IR") refers to formulations or dosage units that rapidly dissolve *in vitro* and are intended to be completely dissolved and absorbed in the stomach or upper gastrointestinal tract. Conventionally, such formulations release at least 90% of the active ingredient within 30 minutes of administration.

"Sustained release" ("SR") refers to formulations or dosage units used herein that are slowly and continuously dissolved and absorbed in the stomach and gastrointestinal tract over a period of six hours or more. Preferred sustained release formulations are those exhibiting plasma concentrations of ranolazine suitable for no more than twice daily administration with two or less tablets per dosing as described below.

"Intravenous (IV) infusion" or "intravenous administration" refers to solutions or dosage units used herein that are provided to the patient by intravenous route. Such IV infusions can be provided to the patient until for up to 96 hours in order to stabilize the patient's cardiovascular condition. The method and timing for delivery of an IV infusion is within the skill of the attending medically trained person.

### 2. Methods of the Invention

In patients with a coronary disease, such as heart failure, it is believed that increased cardiac volume and increased pressure in the heart result in increased levels of natriuretic peptides. As stated above, the invention is directed to a method of identifying the level of natriuretic peptides, such as brain-type natriuretic peptide (BNP) and N-terminal pro-brain natriuretic peptide (NT-proBNP), in the blood plasma of a mammalian patient and, if the level of BNP is elevated, administering ranolazine to the patients. Disclosed are methods for reducing the risk of adverse coronary events including, coronary artery disease (CAD), cardiovascular death, myocardial infarction, acute heart failure, ischemia, recurrent ischemia, acute coronary syndrome, diastolic dysfunction, in patients with a plasma BNP of 80 pg/mL or greater comprising administration of ranolazine.

In the first step a patient is identified that has elevated levels of a natriuretic peptide. Preferably, the levels of BNP and/or NT-proBNP is tested. Preferably, levels of NT-proBNP is tested as it tends to have a longer plasma half life and better *in vitro* stability than the chemically active BNP.

The testing of the peptides can take place in the hospital, the doctor's office, or other suitable environment as is typical in the industry for care of patients. Typically, a patient is asked to rest for 10 to 15 minutes prior to drawing between 5 cc and 25 cc of blood. For most natriuretic peptide testing, the blood is placed in a tube with a chelating agent, such as EDTA, and then centrifuged at below room temperatures. The blood plasma is then separated and run through an immunoassay. The assay provides data relating to the concentration of natriuretic peptide in the blood plasma.

A patient is suitable for treatment by the methods of this invention if the patient exhibits elevated levels of natriuretic peptide as defined above. For heart failure typically, the following schedule dictates the severity of the patient's cardiac condition:
- BNP levels below 100 pg/mL indicate little or no risk of heart failure.
- BNP levels of 100-300 pg/mL suggest a risk of heart failure is present.
- BNP levels above 300 pg/mL indicate a risk of mild heart failure.
- BNP levels above 600 pg/mL indicate a risk of moderate heart failure.
- BNP levels above 900 pg/mL indicate a risk of severe heart failure.

There are several tests available to one of skill in the art to measure these levels. For example, Elecsys® proBNP (Roche, Basel, Switzerland) test can rapidly test for BNP levels. Another example of the assay is the ADVIA® Centaur BNP Assay (Bayer Diagnostics Corporation, Tarrytown, New York), which can rapidly test for BNP levels. Yet another example of the BNP assay is the Triage® BNP Test (Biosite Inc., San Diego, California).

After the patient is identified to have elevated natriuretic peptide levels, the clinician can then administer ranolazine according to the discussion below.

### 3. Compositions of the Invention

As mentioned above, the methods of this invention employ either an IV solution of ranolazine and/or an oral dose of ranolazine. Both of these formulations are discussed below.

### Oral Dose

In one embodiment, the ranolazine is administered an oral dose. In one embodiment, an rural formulation of ranolazine is a tablet. The tablet can be formulated as an instant release or a sustained release tablet. In one embodiment, the tablet of ranolazinc is up to 750 mg ranolazine. In another embodiment, the tablet of ranolazine is up to 1000 mg ranolazine. In a preferred embodiment, the ranolazine tablet is 375 mg ranolazine, and/or 500 mg ranolazine. In another preferred embodiment, the ranolazine tablet is 250 mg ranolazine.

The oral formulation of ranolazine is thoroughly discussed in U.S. Patent No. 6,303,607 and U.S. Publication No. 2003/0220344.

The oral sustained release ranolazine dosage formulations of this invention are administered one, twice, or three times in a 24 hour period in order to maintain a plasma ranolazine level above the threshold therapeutic level and below the maximally tolerated levels, which is preferably a plasma level of 550 to 7500 ng base/mL in a patient.

In a preferred embodiment, the plasma level of ranolazine ranges 1500 to 3500 ng base/mL in a patient.

In order to achieve the preferred plasma ranolazine level, it is preferred that the oral ranolazine dosage forms described herein are administered once or twice daily. If the dosage forms are administered twice daily, then it is preferred that the oral ranolazine dosage forms are administered at twelve hour intervals.

In addition to formulating and administering oral sustained release dosage forms of this invention in a manner that controls the plasma ranolazine levels, it is also important to minimize the difference between peak and trough plasma ranolazine levels. The peak plasma ranolazine levels are typically achieved at from 30 minutes to eight hours or more after initially ingesting the dosage form while trough plasma ranolazine levels are achieved at the time of ingestion of the next scheduled dosage form. It is preferred that the sustained release dosage forms of this invention are administered in a manner that allows for a peak ranolazine level no more than 8 times greater than the trough ranolazine level, preferably no more than 4 times greater than the trough ranolazine level, preferably no more than 3 times greater than the trough ranolazine level, and most preferably no greater than 2 times trough ranolazine level.

The sustained release ranolazine formulations of this invention provide the therapeutic advantage of minimizing variations in ranolazine plasma concentration while permitting, at most, twice-daily administration. The formulation may be administered alone, or (at least initially) in combination with an immediate release formulation if rapid achievement of a therapeutically effective plasma concentration of ranolazine is desired or by soluble IV formulations and oral dosage forms.

### Intravenous Solution

In one aspect, the invention employs an intravenous (IV) solution comprising a selected concentration of ranolazine. Specifically, the IV solution preferably comprises 1.5 to 3.0 mg of ranolazine per milliliter of a pharmaceutically acceptable aqueous solution, more preferably 1.8 to 2.2 mg and even more preferably 2 mg. In order to allow for the rapid intravenous flow of ranolazine into the patient, the IV solution preferably contains no viscous components including by way of example as propylene glycol or polyethylene glycol (e.g., polyethylene glycol 400). It is understood that minor amounts of viscous components that do not materially alter the viscosity may be included in the intravenous formulations of this invention. In a particularly preferred embodiment, the viscosity of the IV solution is preferably less than 10 cSt (centistokes) at 20°C. more preferably less than 5 cSt at 20°C and even more preferably less than 2 cSt at 20°C.

In one embodiment, the IV solution comprises 1.5 to 3.0 mg of ranolazine per mL of IV solution; and either 4.8 to 5.0 weight percent dextrose or 0.8 to 1.0 weight percent sodium chloride.

In one embodiment, the IV solution comprises 1.8 to 2.2 mg of ranolazine per mL of IV solution, and either 4.8 to 5.0 weight percent dextrose or 0.8 to 1.0 weight percent sodium chloride.

In one embodiment, the IV solution of this invention comprises 2 mg of ranolazine per mL of IV solution; and either 4.8 to 5.0 weight percent dextrose or 0.9 weight percent sodium chloride.

The IV solutions described herein can be prepared from a stock solution comprising a 20 mL container for single use delivery which container comprises a sterile aqueous solution of ranolazine at a concentration of 25 mg/mL; either 36 mg/mL dextrose monohydrate or 0.9 weight percent sodium chloride; and having a pH of 4. Surprisingly, employing such high concentrations of ranolazine and dextrose monohydrate or ranolazine and sodium chloride in the stock solutions provide for compositions which are stable and have adequate shelf-lives, preferably of greater than 6 months.

Exemplary methods for preparing the stock solutions are described in Examples 1 and 2.

In a typical setting, two 20 mL containers described herein are injected into an IV container containing 460 mL of sterile saline (0.9 weight percent (w%) sodium chloride) or an aqueous dextrose solution (water containing 5 weight percent dextrose monohydrate) to provide for an IV solution of 2 mg/mL of nanolazine maintained at physiologically acceptable pH. Containers useful herein include, to, vials, syringes, bottles, IV bags.

In another embodiment, the intravenous formulation as set forth above, is diluted with a sterile diluent prior to use. In one embodiment, the sterile diluent is 5 % dextrose or a 0.9 weight percent saline solution. In one embodiment, the intravenous formulation is further diluted into bags of sterile diluent.

### 4. Kits of the Invention

One aspect of the invention is directed to a kit of parts comprising an assay to test for elevated levels of the natriuretic peptide and a pharmaceutical dosage of ranolazine.

The assay may be any of the assays discussed above. The pharmaceutical dosage of ranolazine may be either in the form of an IV solution or an oral dose or both. The IV solution can be packaged in a container as described above. The oral dose is preferably one or two compressed tablets as described above.

### EXAMPLES

Unless stated otherwise, the following definitions are used in the examples.
- µm =: micrometers
- cc =: cubic centimeters
- EDTA =: Ethylene diamine tetraacetic acid
- g =: gram
- mg =: milligram
- mL =: milliliter
- mm =: millimeter
- N =: normal
- rpm =: revolutions per minute

### EXAMPLE 1

### Method of Preparing IV Solution of Ranolazine

20-mL Type 1 flint vial of Ranolazine Injection filled to deliver 20 mL (at 1, 5, or 25 mg/mL ranolazine concentration).

**Compositions**

| | |
|---|---|
| Ranolazine | 1.0, 5.0, 25.0 mg/mL |
| Dextrose monohydrate | 55.0, 52.0, 36.0 mg/mL |
| Hydrochloric acid | q.s. pH to 4.0 ± 0.2 |
| Sodium hydroxide | q.s. pH to 4.0 ± 0.2 |
| Water for Injection | q.s. |

**Container/Closure System**

| | |
|---|---|
| Vial: | Type 1 Flint, 20-cc, 20-mm finish |
| Stopper: | Rubber, 20-mm, West 4432/50, gray butyl, Teflon coated |
| Seal: | Aluminum, 20-mm, flip-top oversea |

### Method of Manufacture

The intravenous formulation of ranolazine is manufactured via an aseptic fill process as follows. In a suitable vessel, the required amount of dextrose monohydrate was dissolved in Water for Injection (WFI) at about 78% of the final batch weight. With continuous stirring, the required amount of ranolazine was added to the dextrose solution. To facilitate the dissolution of ranolazine, the solution pH was adjusted to a target of 3.88-3.92 with a 0.1 N or 1.0 N HCl solution. Additionally, 1 N NaOH may have been utilized to further adjust the solution to the target pH or 3.88-3.92. After ranolazine was dissolved, the batch was adjusted to the final weight with WFI. Upon confirmation that in-process specifications had been met, the ranolazine-formulated bulk solution was sterilized by sterile filtration through two 0.2 µm sterile filters. Subsequently, the sterile ranolazine-formulated bulk solution was aseptically filled into sterile glass vials and aseptically stoppered with sterile stoppers. The stoppered vials were then scaled with clean flip-top aluminum overseals. The vials then went through a final inspection.

### EXAMPLE 2

### Method of Preparing IV Solution of Ranolazine

20-mL Type 1 flint vial of Ranolazine Injection are filled to deliver 20 mL (25 mg/mL concentration).

**Composition**

| | |
|---|---|
| Ranolazine | 25.0 mg/mL |
| Dextrose monohydrate | 36.0 mg/mL |
| Hydrochloric acid | Adjust pH to 3.3-4.7 |
| Water for Injection | q.s. |

**Container/Closure System**

| | |
|---|---|
| Vial: | Type 1 tubing, untreated, 20-mL, 20-mm finish |
| Stopper: | Rubber, 20-mm, West 4432/50, gray butyl |
| Seal: | Aluminum, 20-mm, blue flip-off overseal |

### Method of Manufacture

Water for Injection (WFI) is charged in a suitable vessel at about 90% of the final batch weight. About 90-95% of the required amount of 5 N HCl is added into the compounding vessel. With continuous stirring, the required amount of ranolazine is slowly added, followed by the addition of dextrose monohydrate into the ranolazine solution. To solubilize ranolazine, the solution pH is adjusted with 5 N HCl solution to a target of 3.9-4.1. The batch is subsequently adjusted to the final weight with WFI. Upon confirmation that in-process specifications have been met, the ranolazine-formulated bulk solution is sterilized by filtration through two redundant 0.22 µm sterilizing filters. The sterile ranolazine-formulated bulk solution is then aseptically filled into 20 mL sterile/depyrogenated vials and aseptically stoppered with sterile/depyrogenated stoppers. The stoppered vials are sealed with clean flip-top aluminum overseals. The sealed vials are terminally sterilized by a validated terminal sterilization cycle at 121.1 °C for 30 minutes. After the terminal sterilization process, the vials go through an inspection. To protect the drug product from light, the vials are individually packaged into carton boxes.

### EXAMPLE 3

### Method of Preparing a Sustained Release Tablet

One sustained release formulation of ranolazine employed in this invention, includes a pH dependent binder and a pH independent binder. This formulation was prepared by combining Ranolazine (7500 g), Eudragit L 100-55 (1000 g), hydroxypropyl methylcellulose (Methocel® E5-source) (200 g), and microcrystalline cellulose (Avicel®) (1060 g) by intimate mixing. The mixed powders were granulated with a solution of sodium hydroxide (40 g) in water (1900 to 2500 g). The granulate was dried and screened, mixed with magnesium stearate (200 g), and compressed for example into tablets weighing 667 mg to achieve a dose of 500 mg of ranolazine free base per tablet. The tablets were spray coated in a 24 inch Accelacota® cylindrical pan coater with OPADRY film coating solution to a 2-4% weight gain. OPADRY film coating solutions are available in a variety of colors from Colorcon (West Point, PA).

The stepwise procedure for preparing this formulation is as follows:
a) Blend together ranolazine, microcrystalline cellulose, methacrylate copolymer (Type C) and hydroxypropyl methyl cellulose using an appropriate blender.
b) Dissolve sodium hydroxide in purified water.
c) Using appropriate granulation equipment, slowly add the sodium hydroxide solution to the blend with constant mixing. Add a further aliquot of water, if necessary.
d) Continue mixing to achieve additional massing. Add a further aliquot of water, if necessary.
e) Dry granulated in a fluid bed dryer.
f) Screen dried granules through an appropriate mill.
g) Add magnesium stearate to the screened granules and blend together.
h) Pass the granulated material through a chilsonator, if needed.
i) Compress the granules into tablets using appropriately sized tooling.
j) Disperse OPADRY powder in water and film-coat using appropriately sized coating equipment to a typical level of 2-4% by weight.
k) Polish with carnauba wax using a typical level of 0.002-0.003% by weight.

### EXAM PLE 4

### Identifying Elevated Levels of BNP

The patient rests for about 10-15 minutes by sitting or lying. Blood is drawn in 10 mL aliquots and placed in tubes containing EDTA. The samples are centrifuged at about 0 °C to about 10 °C at about 1,000 to 2,000 rpm. The plasma is then removed and tested using Elecsys® proBNP sandwich immunoassay on an Elecsys® 2010 (Roche Diagnostics, Basel Switzerland). Levels of less about 100 pg/mL suggest little or no risk of heart failure is present. Levels between 100 pg/mL and 300 pg/mL suggest a risk of heart failure is present. Levels above 300 pg/mL indicate a risk of mild heart failure. Levels above 600 pg/mL indicate a risk of moderate heart failure. Levels above 900 pg/mL indicate a risk of severe heart failure. Levels above about 80 pg/mL indicate that the patient may be at risk of an adverse coronary event.

### Treating the patients with ranolazine

After identifying a patient with an elevated NT-proBNP level, a clinician administers ranolazine according to the protocol described above.

### EXAMPLE 5

### Effects of Ranolazine in Patients with non-ST Elevation Acute Coronary Syndromes in the MERLIN-TIMI 36 Trial

### Background

Ranolazine is believed to exert anti-ischemic effects through a reduction in myocardial cellular sodium and calcium overload with a consequent reduction in wall stress. B-type natriuretic peptide (BNP) is released in response to increased wall stress and is a potent indicator of risk in acute coronary syndromes (ACS). This study was a prospective evaluation of the interaction between BNP and the effect of ranolazine as part of a randomized, double-blind, placebo-controlled trial.

Patients with a non-ST elevation acute coronary syndrome (NSTEACS), refers to patients with chest discomfort or anginal equivalent occurring at rest, lasting ≥ 10 minutes, and consistent with myocardial ischemia, and the presence of ischemic symptoms (≥5 minutes) at rest within 48 hours of admittance which may include index episode, and having at least one of the following indicators of moderate - high risk:
- Elevated cardiac troponin (above local MI limit) or CK-MB (>ULN)
- ST-depression (horizontal or down-sloping) ≥ 0.1 mV
- Diabetes mellitus (requiring insulin or oral therapy)
- A Risk Score of ≥ 3 wherein one point is assigned for each of the following variables and a total score calculated as the arithmetic sum:
   o Age ≥ 65 years;
   o Known CAD (prior MI, CABG, PCI or angiographic stenosis ≥50%);
   o Three or more cardiac risk factors (DM, elevated cholesterol, hypertension, family history);
- More than one episode of ischemic discomfort at rest in the prior 24 hours;
- Chronic aspirin use in the 7 days preceding onset of symptoms;
- ST segment depression ≥ 0.05 mV; and
- Elevated cardiac troponin or CK-MB.

### Methods

Plasma BNP were measured (ADVIA® BNP) at baseline (N = 4543), 14 days (N = 4079), and the Final Visit (N = 3328) of the patients with non-ST elevation ACS randomized to ranolazine or placebo in the MERLIN-TIMI 36 trial. During the trial 200 mg of ranolazine (or matching placebo) was administered intravenously over I hour, followed by an 80 mg/hr intravenous infusion, which was reduced to 40 mg/hr for patients with an estimated creatinine clearance of less than 30 mL/min (<0.50 mL/s), and was continued for 12 to 96 hours. On or near the completion (about 1 hour before completion) of the infusion, transition from the IV solution to ranolazine extended release (or matching placebo) occurred and ranolazine extended release (or matching placebo) continued orally at a dose of 1000 mg twice daily until the end of the study. [Morrow, et al., J. Am. Med. Assoc'n. 297 (16), 1775-1783, 2007]. Patients were stratified using BNP >80 pg/mL based upon prior work with this assay. The primary endpoint of the trial was a composite of cardiovascular death, myocardial infarction, or recurrent ischemia (CVD/MI/RI).

### Results

Patients with an elevated concentration of BNP (N = 1935) were at significantly higher risk of CVD/MK/RI (26.4% vs. 20.4%, p<0.0001), as well as CVD/MI (16.2% vs. 7.5%, p<0.0001) and CVD alone (9.0% vs. 2.4%, p<0.0001). In patients with BNP>80 pg/mL, ranolazine was associated with a significant reduction in the primary endpoint (HR 0.79; 95%Cl 0.66-0.94, p=0.009) contrasting with the lack of detectable effect in those with a negative BNP result (see Figure 1, p-interaction = 0.052). This was driven by the endpoint component of worsening angina (HR 0.55; 0.35-0.84; p = 0.005 for BNP>80 pg/mL; HR 0.90; 95% CI 0.63-1.28, p=0.55 for BNP ≤80 pg/mL; p-interaction 0.079).

### Conclusions

Elevated BNP is associated with worse outcomes in patients with ACS. The results of this planned exploratory analysis suggest that ranolazine may have enhanced efficacy in recurrent ischemia in patients with elevated BNP.

From the foregoing description, various modifications and changes in the composition and method will occur to those skilled in the art. All such modifications coming within the scope of the appended claims are intended to be included therein.

## Claims

1. Ranolazine for use in a method of reducing the risk of occurrence of adverse coronary events in a mammalian patient, said method comprising:
a. identifying a patient exhibiting 80 picograms or greater of a natriuretic peptide per milliliter of blood; and
b. administering to said patient a therapeutically effective amount of ranolazine.

2. The ranolazine for use in a method of reducing the risk of occurrence of adverse coronary events in a mammalian patient of claim 1, wherein the adverse coronary events are selected from the group consisting of coronary artery disease (CAD), cardiovascular death, myocardial infarction, acute heart failure, ischemia, recurrent ischemia, acute coronary syndrome, and diastolic dysfunction.

3. The ranolazine for use in a method of reducing the risk of occurrence of adverse coronary events in a mammalian patient of claim 2, wherein the adverse coronary event is acute coronary syndrome, cardiovascular death, myocardial infarction, or recurrent ischemia.

4. The ranolazine for use in a method of reducing the risk of occurrence of adverse coronary events in a mammalian patient of claim 1, wherein the natriuretic peptide is brain-type natriuretic peptide (BNP) or N-terminal pro-brain natriuretic peptide (NT-proBNP).

5. The ranolazine for use in a method of reducing the risk of occurrence of adverse coronary events in a mammalian patient of claim 1, wherein the patient is selected by performing a BNP assay.

6. The ranolazine for use in a method of reducing the risk of occurrence of adverse coronary events in a mammalian patient of claim 1, wherein the patient exhibits between 100 and 300 picograms of natriuretic peptide per milliliter of blood, or between 300 and 600 picograms of natriuretic peptide per milliliter of blood, or between 600 and 900 picograms of natriuretic peptide per milliliter of blood, or greater than 900 picograms of natriuretic peptide per milliliter of blood.

7. The ranolazine for use in a method of reducing the risk of occurrence of adverse coronary events in a mammalian patient of claim 1, wherein the ranolazine is to be administered in an oral dose or as an intravenous (IV) solution.

8. The ranolazine for use in a method of reducing the risk of occurrence of adverse coronary events in a mammalian patient of claim 7, wherein the oral dose is a sustained release tablet.

9. The ranolazine for use in a method of reducing the risk of occurrence of adverse coronary events in a mammalian patient of claim 8, wherein the patient is to be administered the tablet once a day, twice a day, or three times a day.

10. The ranolazine for use in a method of reducing the risk of occurrence of adverse coronary events in a mammalian patient of claim 8, wherein the tablet comprises from 350 to 1000 milligrams of ranolazine.

11. The ranolazine for use in a method of reducing the risk of occurrence of adverse coronary events in a mammalian patient of claim 10, wherein the tablet comprises at least 50% by weight ranolazine, a pH dependent binder, and a pH independent binder, preferably comprises at least 50% by weight ranolazine, from 5 to 12.5% by weight methacrylic acid copolymer, and from 1 to 3% by weight of hydroxypropyl methylcellulose, microcrystalline cellulose, sodium hydroxide, and magnesium stearate.

12. The ranolazine for use in a method of reducing the risk of occurrence of adverse coronary events in a mammalian patient of claim 7, wherein the IV solution comprises from 1.5 to 3 milligrams of ranolazine per milliliter of solution.

13. The ranolazine for use in a method of reducing the risk of occurrence of adverse coronary events in a mammalian patient of claim 12, wherein the IV solution is to be administered to the patient for a time sufficient to reduce the risk of adverse coronary events in the patient.

14. The ranolazine for use in a method of reducing the risk of occurrence of adverse coronary events in a mammalian patient of claim 13, wherein the IV solution is to be administered at 200 mg intravenously over 1 hour.

15. The ranolazine for use in a method of reducing the risk of occurrence of adverse coronary events in a mammalian patient of claim 14, wherein the IV solution is further to be administered as an infusion of 80 mg/hour.

16. The ranolazine for use in a method of reducing the risk of occurrence of adverse coronary events in a mammalian patient of claim 13, wherein the IV solution is to be administered for up to 96 hours.

17. The ranolazine for use in a method of reducing the risk of occurrence of adverse coronary events in a mammalian patient of claim 13, wherein (i) after administration of the IV solution to the patient or (ii) 1 hour prior to completion of the administration of the IV solution, the patient is to be transitioned from the IV solution to an oral dose by administering an oral sustained release formulation of ranolazine.

18. The ranolazine for use in a method of reducing the risk of occurrence of adverse coronary events in a mammalian patient of claim 17, wherein at the time of transition from the IV solution to the oral sustained release formulation of ranolazine in (i), the IV solution is administering 60 mg/hour of ranolazine and the oral dose of ranolazine is 375 mg twice daily, or the IV solution is administering 80 mg/hour of ranolazine and the oral dose of ranolazine is 1000 mg twice daily.

19. The ranolazine for use in a method of reducing the risk of occurrence of adverse coronary events in a mammalian patient of claim 13, wherein the IV solution of ranolazine is to be administered intravenously at 200 mg for 1 hour, followed by an IV infusion of 80 mg/hour for 12 to 96 hours.

20. The ranolazine for use in a method of reducing the risk of occurrence of adverse coronary events in a mammalian patient of claim 19, wherein the patient is to be transitioned from IV solution to the oral sustained release dose of ranolazine, and the oral dose of ranolazine is 1000 mg twice daily.

21. A kit of parts comprising:
a) an assay that detects levels of natriuretic peptides in blood plasma and/or heart tissue of a mammalian patient, and
b) a pharmaceutical dosage of ranolazine.

22. The kit of claim 21, wherein the pharmaceutical dosage of ranolazine is an IV solution or an oral dose, or is both an IV solution and an oral dose.

## Patentansprüche

1. Ranolazin zur Verwendung in einem Verfahren für eine Verringerung des Risikos eines Auftretens von ungünstigen koronaren Ereignissen bei einem Säugerpatienten, wobei das Verfahren umfasst:
a. Identifizieren eines Patienten, der 80 Pikogramm oder mehr eines natriuretischen Peptids pro Milliliter Blut aufweist, und
b. Verabreichen einer therapeutisch wirksamen Menge von Ranolazin an den Patienten.

2. Ranolazin zur Verwendung in einem Verfahren für eine Verringerung des Risikos eines Auftretens von ungünstigen koronaren Ereignissen bei einem Säugerpatienten nach Anspruch 1, wobei die ungünstigen koronaren Ereignisse ausgewählt sind aus der Gruppe, bestehend aus koronarer Arterienkrankheit (CAD), kardiovaskulärem Tod, Myokardinfarkt, akuter Herzinsuffizienz, Ischämie, rezidivierender Ischämie, akutem Koronarsyndrom und diastolischer Funktionsstörung.

3. Ranolazin zur Verwendung in einem Verfahren für eine Verringerung des Risikos eines Auftretens von ungünstigen koronaren Ereignissen bei einem Säugerpatienten nach Anspruch 2, wobei das ungünstige koronare Ereignis akutes Koronarsyndrom, kardiovaskulärer Tod, Myokardinfarkt oder rezidivierende Ischämie ist.

4. Ranolazin zur Verwendung in einem Verfahren für eine Verringerung des Risikos eines Auftretens von ungünstigen koronaren Ereignissen bei einem Säugerpatienten nach Anspruch 1, wobei das natriuretische Peptid Gehirn-typ natriuretisches Peptid (BNP) oder N-terminales pro-Gehirn natriuretisches Peptid (NT-pro BNP) ist.

5. Ranolazin zur Verwendung in einem Verfahren für eine Verringerung des Risikos eines Auftretens von ungünstigen koronaren Ereignissen bei einem Säugerpatienten nach Anspruch 1, wobei der Patient durch Durchführen eines BNP-Tests ausgewählt wird.

6. Ranolazin zur Verwendung in einem Verfahren für eine Verringerung des Risikos eines Auftretens von ungünstigen koronaren Ereignissen bei einem Säugerpatienten nach Anspruch 1, wobei der Patient zwischen 100 und 300 Pikogramm natriuretisches Peptid pro Milliliter Blut oder zwischen 300 und 600 Pikogramm natriuretisches Peptid pro Milliliter Blut oder zwischen 600 und 900 Pikogramm natriuretisches Peptid pro Milliliter Blut oder mehr als 900 Pikogramm natriuretisches Peptid pro Milliliter Blut aufweist.

7. Ranolazin zur Verwendung in einem Verfahren für eine Verringerung des Risikos eines Auftretens von ungünstigen koronaren Ereignissen bei einem Säugerpatienten nach Anspruch 1, wobei das Ranolazin in einer oralen Dosis oder als eine intravenöse (IV) Lösung zu verabreichen ist.

8. Ranolazin zur Verwendung in einem Verfahren für eine Verringerung des Risikos eines Auftretens von ungünstigen koronaren Ereignissen bei einem Säugerpatienten nach Anspruch 7, wobei die orale Dosis eine Tablette mit anhaltender Freisetzung ist.

9. Ranolazin zur Verwendung in einem Verfahren für eine Verringerung des Risikos eines Auftretens von ungünstigen koronaren Ereignissen bei einem Säugerpatienten nach Anspruch 8, wobei die Tablette dem Patienten einmal am Tag, zweimal am Tag oder dreimal am Tag zu verabreichen ist.

10. Ranolazin zur Verwendung in einem Verfahren für eine Verringerung des Risikos eines Auftretens von ungünstigen koronaren Ereignissen bei einem Säugerpatienten nach Anspruch 8, wobei die Tablette 350 bis 1000 Milligramm Ranolazin umfasst.

11. Ranolazin zur Verwendung in einem Verfahren für eine Verringerung des Risikos eines Auftretens von ungünstigen koronaren Ereignissen bei einem Säugerpatienten nach Anspruch 10, wobei die Tablette mindestens 50 Gewichtsprozent Ranolazin, ein pH-abhängiges Bindemittel und ein pH-unabhängiges Bindemittel umfaßt, vorzugsweise mindestens 50 Gewichtsprozent Ranolazin, 5 bis 12,5 Gewichtsprozent Methacrylsäure-Copolymer und 1 bis 3 Gewichtsprozent Hydroxypropylmethylcellulose, mikrokristalline Cellulose, Natriumhydroxid und Magnesiumstearat umfasst.

12. Ranolazin zur Verwendung in einem Verfahren für eine Verringerung des Risikos eines Auftretens von ungünstigen koronaren Ereignissen bei einem Säugerpatienten nach Anspruch 7, wobei die IV-Lösung 1,5 bis 3 Milligramm Ranolazin pro Milliliter Lösung umfasst.

13. Ranolazin zur Verwendung in einem Verfahren für eine Verringerung des Risikos eines Auftretens von ungünstigen koronaren Ereignissen bei einem Säugerpatienten nach Anspruch 12, wobei die IV-Lösung an den Patienten für eine Zeitspanne zu verabreichen ist, die ausreichend ist, um das Risiko für ungünstige koronare Ereignisse bei dem Patienten zu vorringern.

14. Ranolazin zur Verwendung in einem Verfahren für eine Verringerung des Risikos eines Auftretens von ungünstigen koronaren Ereignissen bei einem Säugerpatienten nach Anspruch 13, wobei die IV-Lösung bei 200 Milligramm intravenös über eine Stunde zu verabreichen ist.

15. Ranolazin zur Verwendung in einem Verfahren für eine Verringerung des Risikos eines Auftretens von ungünstigen koronaren Ereignissen bei einem Säugerpatienten nach Anspruch 14, wobei die IV-Lösung ferner als eine Infusion von 80 Milligramm/Stunde zu verabreichen ist.

16. Ranolazin zur Verwendung in einem Verfahren für eine Verringerung des Risikos eines Auftretens von ungünstigen koronaren Ereignissen bei einem Säugerpatienten nach Anspruch 13, wobei die IV-Lösung für bis zu 96 Stunden zu verabreichen ist.

17. Ranolazin zur Verwendung in einem Verfahren für eine Verringerung des Risikos eines Auftretens von ungünstigen koronaren Ereignissen bei einem Säugerpatienten nach Anspruch 13, wobei (i) nach Verabreichung der IV-Lösung an den Patienten oder (ii) eine Stunde vor Abschluss der Verabreichung der IV-Lösung der Patient von der IV-Lösung auf eine orale Dosis durch Verabreichung einer oralen Formulierung mit anhaltender Freisetzung von Ranolazin umzustellen ist.

18. Ranolazin zur Verwendung in einem Verfahren für eine Verringerung des Risikos eines Auftretens von ungünstigen koronaren Ereignissen bei einem Säugerpatienten nach Anspruch 17, wobei bei dem Zeitpunkt der Umstellung von der IV-Lösung auf die orale Formulierung mit anhaltender Freisetzung von Ranolazin in (i) die IV-Lösung eine Verabreichung von 60 Milligramm pro Stunde von Ranolazin ist und die orale Dosis von Ranolazin 375 Milligramm zweimal am Tag ist oder die IV-Lösung eine Verabreichung von 80 Milligramm pro Stunde von Ranolazin ist und die orale Dosis von Ranolazin 1000 Milligramm zweimal am Tag ist.

19. Ranolazin zur Verwendung in einem Verfahren für eine Verringerung des Risikos eines Auftretens von ungünstigen koronaren Ereignissen bei einem Säugerpatienten nach Anspruch 13, wobei die IV-Lösung von Ranolazin intravenös bei 200 Milligramm für eine Stunde, gefolgt von einer IV-Infusion von 80 Milligramm pro Stunde für 12 bis 96 Stunden zu verabreichen ist.

20. Ranolazin zur Verwendung in einem Verfahren für eine Verringerung des Risikos eines Auftretens von ungünstigen koronaren Ereignissen bei einem Säugerpatienten nach Anspruch 19, wobei der Patient von einer IV-Lösung auf die orale Dosis mit anhaltender Freisetzung von Ranolazin umzustellen ist und die orale Dosis von Ranolazin 1000 Milligramm zweimal am Tag ist.

21. Kit, der umfasst:
a) einen Test, der Spiegel von natriuretischen Peptiden in Blutplasma und/oder Herzgewebe eines Säugerpatienten nachweist, und
b) eine pharmazeutische Dosis von Ranolazin.

22. Kit nach Anspruch 21, wobei die pharmazeutische Dosis von Ranolazin eine IV-Lösung oder eine orale Dosis oder sowohl eine IV-Lösung und eine orale Dosis ist.

## Revendications

1. Ranolazine pour utilisation dans un procédé pour la réduction du risque d'occurrence d'événements coronaires indésirables chez un patient mammalien, ledit procédé comprenant:
a. l'identification d'un patient présentant 80 picogrammes ou plus d'un peptide natriurétique par millilitre de sang; et
b. l'administration audit patient d'une quantité de ranolazine thérapeutiquement efficace.

2. Ranolazine pour utilisation dans un procédé pour la réduction du risque d'occurrence d'événements coronaires indésirables chez un patient mammalien selon la revendication 1, où les événements coronaires indésirables sont choisis dans le groupe consistant en insuffisance coronarienne (CAD), mort cardiovasculaire, infarctus du myocarde, insuffisance cardiaque aigue, ischémie, ischémie récurrente, syndrome coronaire aigu, et dysfonctionnement diastolique.

3. Ranolazine pour utilisation dans un procédé pour la réduction du risque d'occurrence d'événements coronaires indésirables chez un patient mammalien selon la revendication 2, où l'événement coronaire indésirable est le syndrome coronaire aigu, la mort cardiovasculaire, l'infarctus du myocarde, ou l'ischémie récurrente.

4. Ranolazine pour utilisation dans un procédé pour la réduction du risque d'occurrence d'événements coronaires indésirables chez un patient mammalien selon la revendication 1, où le peptide natriurétique est le peptide natriurétique cérébral (BNP) ou le peptide natriurétique pro-cérébral N-terminal (NT-pro-BNP).

5. Ranolazine pour utilisation dans un procédé pour la réduction du risque d'occurrence d'événements coronaires indésirables chez un patient mammalien selon la revendication 1, où le patient est choisi pour la réalisation d'un essai de BNP.

6. Ranolazine pour utilisation dans un procédé pour la réduction du risque d'occurrence d'événements coronaires indésirables chez un patient mammalien selon la revendication 1, où le patient présente entre 100 et 300 picogrammes de peptide natriurétique par millilitre de sang, ou entre 300 et 600 picogrammes de peptide natriurétique par millilitre de sang, ou entre 600 et 900 picogrammes de peptide natriurétique par millilitre de sang, ou plus de 900 picogrammes de peptide natriurétique par millilitre de sang.

7. Ranolazine pour utilisation dans un procédé pour la réduction du risque d'occurrence d'événements coronaires indésirables chez un patient mammalien selon la revendication 1, où la ranolazine est destinée à être administrée en une dose orale ou sous forme d'une solution intraveineuse (IV).

8. Ranolazine pour utilisation dans un procédé pour la réduction du risque d'occurrence d'événements coronaires indésirables chez un patient mammalien selon la revendication 7, où la dose orale est un comprimé à libération prolongée.

9. Ranolazine pour utilisation dans un procédé pour la réduction du risque d'occurrence d'événements coronaires indésirables chez un patient mammalien selon la revendication 8, où le patient est destiné à recevoir l'administration du comprimé une fois par jour, deux fois par jour, ou trois fois par jour.

10. Ranolazine pour utilisation dans un procédé pour la réduction du risque d'occurrence d'événements coronaires indésirables chez un patient mammalien selon la revendication 8, où le comprimé comprend entre 350 et 1000 milligrammes de ranolazine.

11. Ranolazine pour utilisation dans un procédé pour la réduction du risque d'occurrence d'événements coronaires indésirables chez un patient mammalien selon la revendication 10, où le comprimé comprend au moins 50% en poids de ranolazine, un liant dépendant du pH, et un liant indépendant du pH, de préférence comprend au moins 50% en poids de ranolazine, de 5 à 12,5% en poids de copolymère d'acide méthacrylique, et de 1 à 3% en poids d'hydroxypropyl méthylcellulose, de cellulose microcristalline, d'hydroxyde de sodium, et de stéarate de magnésium.

12. Ranolazine pour utilisation dans un procédé pour la réduction du risque d'occurrence d'événements coronaires indésirables chez un patient mammalien selon la revendication 7, où la solution IV comprend de 1,5 à 3 milligrammes de ranolazine par millilitre de solution.

13. Ranolazine pour utilisation dans un procédé pour la réduction du risque d'occurrence d'événements coronaires indésirables chez un patient mammalien selon la revendication 12, où la solution IV est destinée à être administrée au patient pendant une durée suffisante pour réduire le risque d'événements coronaires indésirables chez le patient.

14. Ranolazine pour utilisation dans un procédé pour la réduction du risque d'occurrence d'événements coronaires indésirables chez un patient mammalien selon la revendication 13, où la solution IV est destinée à être administrée à raison de 200 mg par voie intraveineuse en 1 heure.

15. Ranolazine pour utilisation dans un procédé pour la réduction du risque d'occurrence d'événements coronaires indésirables chez un patient mammalien selon la revendication 14, où la solution IV est en outre destinée à être administrée sous forme d'une perfusion de 80 mg/heure.

16. Ranolazine pour utilisation dans un procédé pour la réduction du risque d'occurrence d'événements coronaires indésirables chez un patient mammalien selon la revendication 13, où la solution IV est destinée à être administrée pendant jusqu'à 96 heures.

17. Ranolazine pour utilisation dans un procédé pour la réduction du risque d'occurrence d'événements coronaires indésirables chez un patient mammalien selon la revendication 13, où (i) après l'administration de la solution IV au patient ou (ii) une heure avant l'achèvement de l'administration de la solution IV, le patient est destiné à être passé de la solution IV à une dose orale par administration d'une formulation orale à libération prolongée de ranolazine.

18. Ranolazine pour utilisation dans un procédé pour la réduction du risque d'occurrence d'événements coronaires indésirables chez un patient mammalien selon la revendication 17, où au moment de la transition de la solution IV à la formulation orale à libération prolongée de ranolazine dans (i), la solution IV est administrée à raison de 60 mg/heure de ranolazine et la dose orale de ranolazine est de 375 mg deux fois par jour, ou la solution IV est administrée à raison de 80 mg/heure de ranolazine et la dose orale de ranolazine est de 1000 mg deux fois par jour.

19. Ranolazine pour utilisation dans un procédé pour la réduction du risque d'occurrence d'événements coronaires indésirables chez un patient mammalien selon la revendication 13, où la solution IV de ranolazine est destinée à être administrée par voie intraveineuse à raison de 200 mg pendant 1 heure, suivie par une perfusion IV de 80 mg/heure pendant 12 à 96 heures.

20. Ranolazine pour utilisation dans un procédé pour la réduction du risque d'occurrence d'événements coronaires indésirables chez un patient mammalien selon la revendication 19, où le patient est destiné à être passé d'une solution IV à la dose orale à libération prolongée de ranolazine, et la dose orale de ranolazine est de 1000 mg deux fois par jour.

21. Kit de parties comprenant:
a) un essai qui détecte les niveaux de peptides natriurétiques dans le plasma sanguin et/ou le tissu cardiaque d'un patient mammalien, et
b) un dosage pharmaceutique de ranolazine.

22. kit selon la revendication 21, où le dosage pharmaceutique de ranolazine est une solution IV ou une dose orale, ou il est à la fois une solution IV et une dose orale.
